# EUROPEAN PATENT SPECIFICATION

(11) **EP 4 620 438 B1**
(45) Date of publication and mention of the grant of the patent: **28.10.2026**
(21) Application number: 25185337.0
(22) Date of filing: 22.07.2021
(51) Int. Cl.: A61F 5/445

(54) **DOUBLE-SIDED SENSOR DEVICE FOR OSTOMY LEAK DETECTION SYSTEM**
DOPPELSEITIGE SENSORVORRICHTUNG FÜR EIN STOMA-LECKERKENNUNGSSYSTEM
DISPOSITIF DE CAPTEUR DOUBLE FACE POUR SYSTÈME DE DÉTECTION DE FUITE DE STOMIE

(30) Priority: 24.07.2020 US 202063056289 P
(43) Date of publication of application: 24.09.2025
(62) Document number(s) of the earlier application(s) in accordance with Art. 76 EPC: 21752854.6 / 4 185 252
(73) Proprietor: Hollister Incorporated, Libertyville, IL 60048 (US)
(72) Inventor: CARLSSON, Jonas P., Libertyville, 60048 (US); LATTANZI, Lauren M., Libertyville, 60048 (US); MATTHEWS, Kyle A., Libertyville, 60048 (US)
(74) Representative: FRKelly

(56) References cited:
- WO-A1-2007/098762
- WO-A1-2019/120424
- WO-A1-2020/076609

## Description

### BACKGROUND

The following description relates generally to a leakage detection system for medical devices, and more particularly to a sensor device for detecting leakage in ostomy appliances.

An ostomy pouch system typically includes a pouch formed from opposing sidewalls defining an internal collection area, an inlet opening for receiving a stoma, and an ostomy appliance for attaching the pouch to a user. The ostomy appliance may include, for example, an ostomy barrier of a one-piece pouch system, which is attached to one of the pouch sidewalls proximate an inlet opening, a faceplate for a two-piece pouch system configured to releasably engage a pouch, and a barrier ring. The ostomy appliance may include a skin barrier material for adhering to and sealing against user's peristomal skin surrounding the stoma.

The ostomy appliance may be susceptible to ostomy effluent leakage, and the seal formed between the skin barrier material and the user may weaken. Often times, the user may be unaware of or cannot easily assess an extent of weakening in the seal. Thus, the user may not become aware of a weakened seal, and consequently, the ostomy effluent may leak through to an exterior of the ostomy appliance.

WO2007098762A1 discloses a method and dressing for detecting detachment of the dressing, which is applied to a surface of an at least partly electrically conductive object. The dressing comprises an adhesive for attaching the dressing to the electrically conductive object and at least two electrodes arranged in a distance from the electrically conductive object. A voltage is applied to the first and second electrode establishing an electrical circuit comprising a first capacitor between the first electrode and the electrically conductive object and a second capacitor between the second electrode and the electrically conductive object. Changes of the capacitance between at least one of the first and the second electrode and the electrically conductive object are detected, and an alarm is activated when the changes of the capacitance reach a predetermined value. This advantageously provides a method whereby a leak can be detected quickly.

WO2019120424A1 discloses a system for determining and signalling moisture propagation in an adhesive material layer of a base plate and/or a sensor assembly part for an ostomy appliance. The disclosure further relates to aspects of a base plate and/or a sensor assembly part for an ostomy appliance and its use in such a system.

WO2020076609A1 discloses an ostomy appliance system including an ostomy appliance having a stoma opening and a first electrical interface, and at least one thermal sensor configured to detect at least one thermal property, such as temperature, at the ostomy appliance, the at least one temperature sensor connected to the first electrical interface with electrical circuitry. A wearable device may be removably connected to the ostomy appliance and operably connected to the at least one thermal sensor. The wearable device includes a housing, a second electrical interface configured for electrical connection to the first electrical interface, a power supply and a controller operably connected to the power supply. The controller is configured to determine a condition of the ostomy appliance based on the at least one thermal property detected at the at least one thermal sensor. The wearable device may also include a wireless transceiver configured to communicate with a personal communication device.

Accordingly, it is desirable to provide a leakage detection system for ostomy appliances.

### SUMMARY

The present disclosure provides a sensor device as detailed in claim 1. Advantageous features are provided in dependent claims.

In one aspect, a sensor device for detecting ostomy effluent leakage in an ostomy appliance is provided. The sensor device may be provided as an accessory that can be used with an ostomy barrier or a faceplate. The sensor device may include a sensor layer and a skin barrier layer for attaching the sensor device to a user. The sensor layer may include a substrate, a plurality of sensing electrodes arranged on a proximal side of the substrate, a plurality of connecting traces arranged on a distal side of the substrate, and a plurality of connection points configured to electrically connect the sensor device to a separate control unit, such as a wearable device. The skin barrier layer may be arranged adjacent the plurality of sensing electrodes. The plurality of connecting traces may be electrically insulated from the skin barrier layer by the substrate.

In an embodiment, the sensor layer may include a generally ring-shaped sensor region, a connector region, and a tail region connecting the sensor region and the connector region. The plurality of the sensing electrodes may be arranged in the sensor region and the plurality of the connection points may be arranged in the connector region. Each of the sensing electrodes may be electrically connected to at least one of the connection points by at least one of the connecting traces.

In some embodiments, each of the sensing electrodes may be electrically connected to at least one of the connecting traces through a via. The plurality of connection points may be arranged on the proximal side of the substrate, wherein each of the connecting traces may be electrically connected to at least one of the connection points through a via. The vias may be formed from a suitable electrically conductive material and may extend through the thickness of the substrate.

The plurality of sensing electrodes may comprise a first sensing electrode, a second sensing electrode, and a third sensing electrode. The plurality of the connecting traces may comprise a first connecting trace, a second connecting trace, and a third connecting trace. Further, the plurality of connection points may comprise a first connection point, a second connection point, and a third connection point.

In an embodiment, the first sensing electrode may be electrically connected to the first connecting trace through a first via. The second sensing electrode may be electrically connected to the second connecting trace through a second via. The third sensing electrode may be electrically connected to the third connecting trace through a third via. In such an embodiment, the first connecting trace may be electrically connected to the first connection point through a fourth via. The second connecting trace may be electrically connected to the second connection point through a fifth via. The third connecting trace may be electrically connected to the third connection point through a sixth via.

In another embodiment, the first sensing electrode may be electrically connected to the first connecting trace through a first via. The second sensing electrode may be electrically connected to the second connecting trace through a second via, and the third sensing electrode may be electrically connected to the second connecting trace through a third via. In such an embodiment, the first connecting trace may be electrically connected to the first connection point through a fourth via, and the second connecting trace may be electrically connected to the second connection point through a fifth via.

In an embodiment, the first, second, and third sensing electrodes may be substantially circular electrodes, wherein each of the first, second, and third sensing electrodes may be arranged at a different radial distance from a center opening of the sensor device. The first, second, and third sensing electrodes may be configured to determine a radial distance of an ostomy effluent leakage location.

In another embodiment, the first, second, and third sensing electrodes may be arc shaped electrodes, wherein each of the first, second, and third sensing electrodes may be arranged in a different section of the sensor region. The first, second, and third sensing electrodes may be configured to determine a location of an ostomy effluent leakage.

In an embodiment, the plurality of sensing electrodes may further comprise a fourth sensing electrode having an arc shape, and the plurality of connecting traces may further include a fourth connecting trace. The first sensing electrode may be arranged in a first quadrant of the sensor region, the second sensing electrode may be arranged in a second quadrant of the sensor region, the third sensing electrode may be arranged in a third quadrant of the sensor region, and the fourth sensing electrode may be arranged in a fourth quadrant of the sensor region. The sensor device may be configured to determine a location of an ostomy effluent leakage based on the location of the sensing electrode that detected a change in an electrical resistance.

In such an embodiment, the first sensing electrode arranged on the proximal side of the substrate in the first quadrant may be electrically connected to the first connecting trace through a first via, wherein the first connecting trace may extend along the fourth quadrant of the sensor region and along the tail region on the distal side of the substrate. The second sensing electrode arranged on the proximal side of the substrate in the second quadrant may be electrically connected to the second connecting trace through a second via, wherein the second connecting trace may extend along the third quadrant of the sensor region and along the tail region on the distal side of the substrate. The third sensing electrode arranged on the proximal side of the substrate in the third quadrant may be electrically connected to the third connecting trace through a third via, wherein the third connecting trace may extend from a lower portion of the sensor region and along the tail region on the distal side of the substrate. Further, the fourth sensing electrode arranged on the proximal side of the substrate in the fourth quadrant may be electrically connected to the fourth connecting trace through a fourth via, wherein the fourth connecting trace may extend from a lower portion of the sensor region and along the tail region on the distal side of the substrate.

The plurality of connection points may be arranged on the proximal side of the substrate and further comprise a fourth connection point. In such an embodiment, the first connecting trace may be electrically connected to the first connection point via a fifth via. The second connecting trace may be electrically connected to the second connection point via a sixth via. The third connecting trace may be electrically connected to the third connection point via a seventh via, and the fourth connecting trace may be electrically connected to the fourth connection point via an eighth via.

In any of the foregoing embodiments, the sensor device may be configured to detect an ostomy effluent leakage based a change in electrical resistance measured by the plurality of sensing electrodes.

In an embodiment, the control unit may comprise a plurality of electrical connectors configured to interface with the plurality of connection points to electrically connect the control unit to the sensor device. The control unit may be configured to provide an electrical current to at least one of the sensing electrodes and detect a change in electrical resistance measured by the sensing electrodes. In some embodiments, the control unit may be configured as a wearable device that can removably connect to the sensor device.

Other objects, features, and advantages of the disclosure will be apparent from the following description, taken in conjunction with the accompanying sheets of drawings, wherein like numerals refer to like parts, elements, components, steps, and processes.

### BRIEF DESCRIPTION OF THE DRAWINGS

FIG. 1 is a plan view illustrating a body-side of a sensor device according to an embodiment;
FIG. 2 is a plan view illustrating a pouch-side of the sensor device of FIG. 1;
FIG. 3 is an exploded view of the sensor device of FIG. 1;
FIG. 4 is a schematic illustration of a proximal side view of a double-sided sensor layer according to an embodiment;
FIG. 5 is a schematic illustration of a distal side view of the double-sided sensor layer of FIG. 4;
FIG. 6 is an illustration of a control unit according to an embodiment; and
FIG. 7 is an illustration of the control unit of FIG. 6 connected to the sensor device of FIG. 1.

### DETAILED DESCRIPTION

While the present disclosure is susceptible of embodiment in various forms, there is shown in the drawings and will hereinafter be described one or more embodiments with the understanding that the present disclosure is to be considered illustrative only and is not intended to limit the disclosure to any specific embodiment described or illustrated.

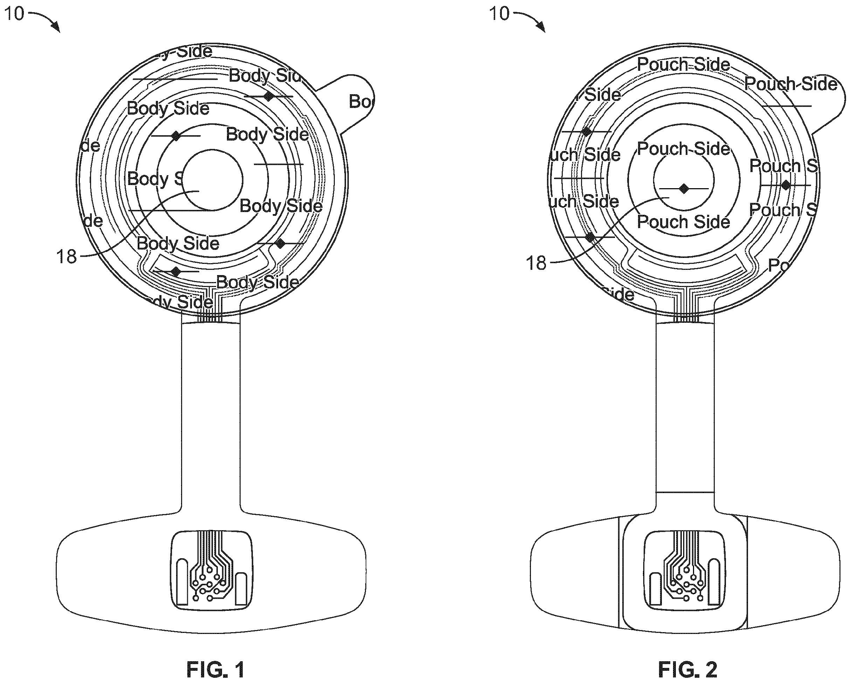

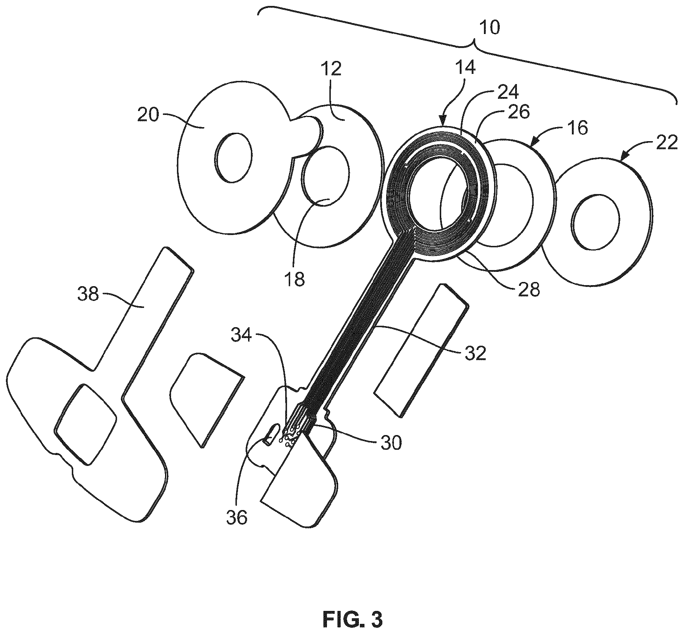

Referring to FIGS. 1-3, a sensor device 10 for an ostomy appliance according to an embodiment is illustrated. The sensor device 10 may be provided as an ostomy accessory, which may be attached to an ostomy barrier or a faceplate configured to secure an ostomy pouch to a user. FIGS. 1 and 2 show a body-side view and a pouch-side view of the sensor device 10. As best shown in FIG. 3, the sensor device 10 may include an adhesive layer 12, a sensor layer 14 and a barrier-side layer 16. A center opening 18 configured to receive a stoma may extend through the sensor device 10. The center opening 18 may be formed by respective openings extending through individual layers of the sensor device 10. Each layer 12, 14, 16 of the sensor device 10 may have a proximal side and a distal side. When the sensor device 10 is attached to a patient, the respective proximal sides generally face toward the patient and the respective distal sides generally face away from the patient.

The adhesive layer 12 may be disposed on the body-side of the sensor device 10. In an embodiment, the proximal side of the adhesive layer 12 may form at least a portion of the body-side of the sensor device 10. The proximal side of the adhesive layer 12 may be configured to adhere to peristomal skin surfaces of the patient and seal around the stoma. The adhesive layer 12 may be formed from a medical-grade pressure sensitive adhesive that can adhesively secure the sensor device 10 to a patient's peristomal skin surfaces. For example, the adhesive layer 12 may be formed from a hydrocolloid adhesive. A release liner 20 may be provided on the proximal side of the adhesive layer 12 to cover the adhesive, which may be removed by a user before attaching the sensor device 10 to user's skin.

The barrier-side layer 16 may be formed from a soft, flexible material that is generally soft and non-irritable to the user's skin, such as an adhesive, polymeric film, nonwoven or foam material. In an embodiment, the barrier-side layer 16 may be formed from an adhesive, such as a hydrocolloid adhesive. In such an embodiment, a release liner 22 may be provided on the distal side of the barrier-side layer 16 to cover the adhesive, which may be removed by a user before applying the sensor device 10 to an ostomy barrier or faceplate.

The sensor layer 14 may include an electrically conductive circuitry 24, such as a plurality of electrodes, conductive traces or the like. The electrically conductive circuitry 24 may be disposed on a substrate 26. In an embodiment, the sensor layer 14 may include a sensor region 28, a connector region 30 and a tail region 32 connecting the sensor region 28 and the connector region 30. The electrically conductive circuitry 24 may be arranged in a predetermined pattern at the sensor region 28. For example, the electrically conductive circuitry 24 may be arranged generally in a circular or semi-circular pattern. Other suitable patterns are envisioned as well, such as an oval or oblong pattern, or other closed or substantially closed loop pattern. The electrically conductive circuitry 24 at the sensor region 28 may be arranged at one or more radial distances from the center opening 18. For example, the electrically conductive circuit 24 may be arranged at a plurality of different, radial distances from the center opening 18.

In an embodiment, the tail region 32 may generally be formed as an elongated section extending from the sensor region 28. The tail region 32 may extend beyond an outer periphery of the first adhesive layer 12 and/or the barrier-side layer 16 in a direction radially outward from the center opening 18. The electrically conductive circuitry 24 may extend along the tail region 32. In an embodiment, the tail region 32 may be flexible along at least a portion of its length such that it may be folded or wrapped.

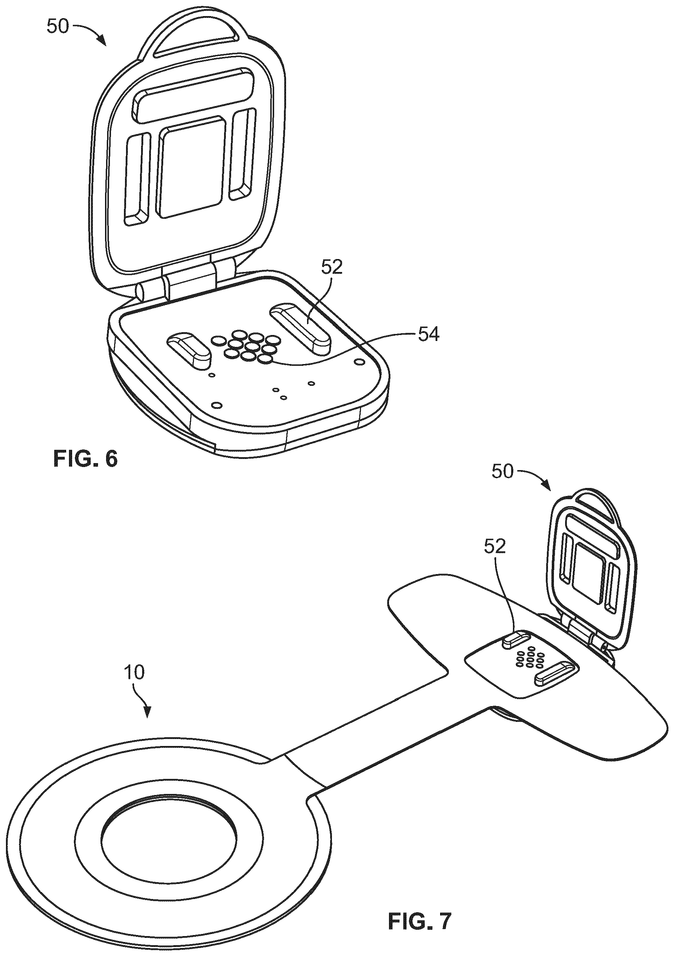

The connector region 30 may include a plurality of connection points 34 electrically connected to the electrically conductive circuitry 24. The connection points 34 may include an externally accessible portion configured for electrical connection to an external device, such as a control unit 50 (FIGS. 6 and 7). The control unit 50 may be, for example, a wearable device. In this manner, the connection points 34 may provide an electrical connection between the control unit 50 and the electrically conductive circuitry 24. The externally accessible portion of the connection points 34 may be any suitable electrical interface for forming an electrical connection between two electrical components, such as one or more electrically conductive contacts, pins, and the like.

The connector region 30 may also include one or more alignment members 36. The one or more alignment members 36 may be configured to engage corresponding alignment members 52 of the control unit 50 to facilitate positioning of the connector region 30 relative to the control unit 50 to provide the electrical connection as shown in FIG. 7. In an embodiment, the one or more alignment members 36 of the connector region 30 may be an opening, recess or slot. The corresponding alignment members 52 of the control unit 50 may be one or more projections configured for receipt in the opening, recess or slot of the connector region 30.

In an embodiment, the sensor device 10 may be configured to detect a leakage by measuring resistance between electrodes. For example, the sensor device 10 may be configured to detect a change in resistance between electrodes triggered by ostomy effluent bridging the electrodes as a leakage propagates. In the embodiment of FIG. 3, the electrically conductive circuitry 24 may comprise a plurality of electrodes arranged on the proximal side of the sensor region 28, such that the electrodes may be positioned adjacent and in contact with the adhesive layer 12 to measure resistance of the adhesive layer 12. The plurality of electrodes 24 may extend along the proximal side of the tail region 32 and along a portion of the connector region 30 to the connection points 34. In such an embodiment, a masking element may be used to prevent shorting between electrodes in the areas where detection is not desired. For example, a masking element 38 may be provided on the body-side of the sensor device 10 to cover the plurality of electrodes 24 in the tail region 32 and the connector region 30.

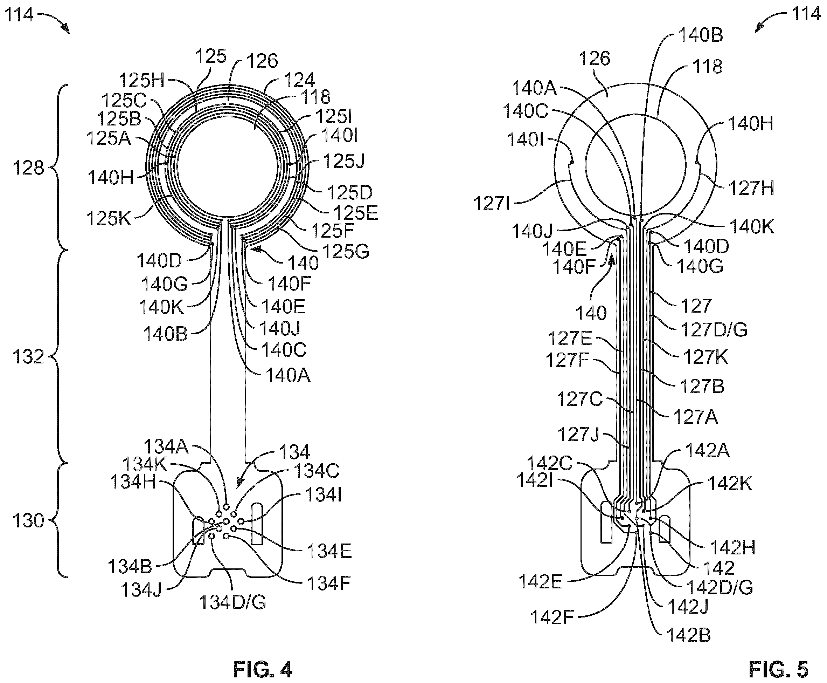

In an embodiment, the sensor layer may be configured as a double-sided sensor layer, wherein at least a portions of the conductive circuitry may be arranged on the proximal side of the sensor layer while another portion of the conductive circuitry is arranged on the distal side the sensor layer. FIGS. 4 and 5 show a proximal side view and a distal side view of a double-sided sensor layer 114 according to an embodiment. The double-sided sensor layer 114 may be configured similar to the sensor layer 14 generally comprising a generally ring-shaped sensor region 128, a connector region 130, a tail region 132 extending between the sensor region 128 and the connector region 130, an electrically conductive circuitry 124, and a plurality of connection points 134 provided on a substrate 126. In this embodiment, at least a portion of the electrically conductive circuitry 124 may be arranged on the proximal side of the substrate 126 while another portion of the electrically conductive circuitry 124 may be arrange on the distal side of the substrate 126. In such an embodiment, the portion of the electrically conductive circuitry 124 arranged on the distal side of the substrate 126 may be electrically insulated from the proximal side of the sensor layer 114 by the substrate 126, such that a masking element may not be necessary on the proximal side of the sensor layer 114.

In an embodiment, the electrically conductive circuitry 124 may include a plurality of sensing electrodes 125 arranged on a proximal side of the sensor layer 114 (FIG. 4) and a plurality of connecting traces 127 arranged on a distal side of the sensor layer 114 (FIG. 5). The plurality of sensing electrodes 125 may be arranged adjacent and in contact with the adhesive layer 12 in the sensor region 128 of the sensor layer 114 and may be configured to detect a change of resistance in the adhesive layer 12. The plurality of connecting traces 127 may be configured to electrically connect the plurality of sensing electrodes 125 to the plurality of connection points 134 arranged in the connection region 130. By arranging the plurality of connecting traces 127, which are non-sensing portions of the electrically conductive circuitry 124, on the distal side of the sensor layer 114, a need for masking elements to electrically insulate the non-sensing portions for accurate leakage detection may be eliminated. In the embodiment of FIGS. 4 and 5, the connection points 134 are arranged on the proximal side of the substrate 126. In other embodiments, the connection points 134 may be arranged on the distal side of the substrate 126.

In some embodiments, the sensor layer 114 may include a plurality of vias 140, 142 configured to provide an electrical connection between the plurality of the sensing electrodes 125 and the plurality of connecting traces 127 and between the plurality of the connecting traces 127 and the plurality of connection points 134. The vias may be formed from a suitable electrically conductive material, such as copper. In such embodiments, each of the sensing electrodes 125, which is arranged on the proximal side of the substrate 126, may be electrically connected to at least one of the plurality of connecting trace 127 arranged on the distal side of the substrate 126 through a via 140 extending through the thickness of the substrate 126. Further, each of the plurality of connecting traces 127, which extends along the distal side of the substrate 126, may be electrically connected to at least one of the plurality of connection points 134 arranged on the proximal side of the substrate 126 through a via 142 that extends through the thickness of the substrate 126.

In the embodiment of FIGS. 4 and 5, the plurality of sensing electrodes 125 may comprise seven substantially circular electrodes, also referred to as "circular electrodes" herein, 125A, 125B, 125C, 125D, 125E, 125F, 125G, and four arc shaped electrodes, also referred to as "electrode arcs" herein, 125H, 125I, 125J, 125K. Each of the circular electrodes 125A-G may be arranged at a different radial distance from a center opening 118 of the sensor layer 114 as shown in FIG. 4 and configured to determine a radial progress of ostomy effluent leakage. The four electrode arcs 125H, 125I, 125J, 125K may be arranged in different sections of the sensor region 128 and configured to determine a location of a leak in the sensor region 128. In this embodiment, a first electrode arc 125H may be arranged to extend along a first quadrant of the sensor region 128, a second electrode arc 125I may be arranged to extend along a second quadrant of the sensor region 128, a third electrode arc 125J may be arranged to extend along a third quadrant of the sensor region 128, and a fourth electrode arc 125K may be arranged to extend along a fourth quadrant of the sensor region 128, such that a change in electrical resistance measured by one of the four electrode arcs may be used to determine the location of a leakage. In other embodiments, the sensor layer 114 may include less than four electrode arcs or more than four electrode arcs, which may be arranged in different sections of the sensor region 128 and configured to determine a location of a leakage.

Each of the plurality of the sensing electrodes 125A-125K may be connected to at least one of the plurality of connecting traces 127 through a via 140. In the embodiment of FIGS. 4 and 5, a first circular electrode 125A may be connected to a first connecting trace 127A through a first via 140A, a second circular electrode 125B may be connected to a second connecting trace 127B through a second via 140B, a third circular electrode 125C may be connected to a third connecting trace 127C through a third via 140C, a fourth circular electrode 125D may be connected to a fourth connecting trace 127D/G through a fourth via 140D, a fifth circular electrode 125E may be connected to a fifth connecting trace 127E through a fifth via 140E, a sixth circular electrode 125F may be connected to a sixth connecting trace 127F through a sixth via 140F, and a seventh circular electrode 125G may be connected to the fourth connecting trace 127D/G through a seventh via 140G. In this embodiment, two sensing electrodes 125D and 125G are connected to a single connecting trace 127 D/G. In some embodiments, more than two sensing electrodes may be connected to a single connecting trace.

As shown in FIG. 4, the first electrode arc 125H may be arranged in the first quadrant or an upper left quadrant of the sensor region 128 (when viewed from the proximal side) to detect a leakage in the first quadrant. The first electrode arc 125H may be connected to a seventh connecting trace 127H through an eighth via 140H. The seventh connecting trace 127H may extend along a fourth quadrant or a lower left quadrant of the sensor region 128 (when viewed from the proximal side) on the distal side and along the tail region 132 to electrically connect the first electrode arc 125H to a connection point 134H. The second electrode arc 125I may be arranged in the second quadrant or an upper right quadrant of the sensor region 128 (when viewed from the proximal side) to detect a leakage in the second quadrant. The second electrode arc 125I may be connected to an eighth connecting trace 127I through a ninth via 140I. The eighth connecting trace 127I may extend along a third quadrant or a lower right quadrant of the sensor region 128 (when viewed from the proximal side) on the distal side and along the tail region 132 to electrically connect the second electrode arc 125I to a connection point 134I. The third electrode arc 125J may be arranged in the third quadrant or a lower right quadrant of the sensor region 128 (when viewed from the proximal side) to detect a leakage in the third quadrant. The third electrode arc 125J may be connected to ninth connecting trace 127J through a tenth via 140J. The fourth electrode arc 125K may be arranged in the fourth quadrant or a lower left quadrant of the sensor region 128 (when viewed from the proximal side) to detect a leakage in the fourth quadrant. The fourth electrode arc 125K may be connected to tenth connecting trace 127K through a 11^{th} via 140K.

The ten connecting traces 127A-K, which are electrically connected to the eleven sensing electrodes 125A-K, may extend from the sensor region 128 along the tail region 132 to the connector region 130 on the distal side of the substrate 126. The connecting traces 127A-K may be electrically connected to the connection points 134A-K through the plurality of vias 142A-K. In the embodiment of FIGS. 4 and 5, the first connecting trace 127A may be connected to a first connection point 134A through a 12^{th} via 142A, the second connecting trace 127B may be connected to a second connection point 134B through a 13^{th} via 142B, the third connecting trace 127C may be connected to a third connection point 134C through a 14^{th} via 142C, the fourth connecting trace 127D/G may be connected to a fourth connection point 134D/G through a 15^{th} via 142D/G, the fifth connecting trace 127E may be connected to a fifth connection point 134E through a 16^{th} via 142E, the sixth connecting trace 127F may be connected to a sixth connection point 134F through a 17^{th} via 142F, the seventh connecting trace 127H may be connected to a seventh connection point 134H through a 18^{th} via 142H, the eighth connecting trace 127I may be connected to an eighth connection point 134I through a 19^{th} via 142I, the ninth connecting trace 127J may be connected to a ninth connection point 134J through a 20^{th} via 142J, and the tenth connecting trace 127K may be connected to a tenth connection point 134K through a 21^{th} via 142K. In this embodiment, the sensor layer 114 may comprise eleven sensing electrodes 125 electrically connected to ten connection points 134 though ten connecting traces 127 and twenty-one vias 140, 142.

In the embodiment of FIGS. 4 and 5, only the sensing electrodes 125 configured to measure resistance may be exposed on the proximal side of the double-sided sensor layer 114, while the connecting traces 127 may be arranged away from the proximal side, for example, on the distal side or between substrates. As such, a masking element for preventing shorting between electrodes or traces may not be necessary on the proximal side of the sensor layer 114. In an embodiment, at least a portion of the sensor layer 114 may be covered with a masking element on the distal side.

The substrate 126 may be formed from a suitable flexible polymeric material, such as a polyimide film. The substrates 126 may have a thickness of about 0.5 mil to about 3 mil, preferably, about 1 mil to about 2 mil, and more preferably about 1.5 mil. In an embodiment, the substrate 126 may be formed from a coverlay film having a thickness of about 1.3 mil and comprising a polyimide film coated with an acrylic adhesive on one side, such as PYRALUX^{®} FR7013 available from Dupont. In some embodiments, the substrate 126 may comprise two or more layers, wherein the connecting traces 127 may be arranged between the substrate layers.

FIGS. 6 shows the control unit 50 according to an embodiment. The control unit 50 may be selectively and removably and electrically connected to the sensor accessory 10 as shown in FIG. 7. For example, the control unit 50 may include a plurality of electrical connectors 54 configured to interface with the plurality of connection points 134. The control unit 50 may be removably connected to the plurality of connection points 134 by a known, suitable mechanical fastener, such as a spring-load clip, mechanical interlock, clamp, interference fit, and the like, including combinations thereof.

The control unit 50, for example via a controller, may be configured to provide an electrical current to the electrically conductive circuitry 124 and detect a change in electrical resistance measured by the sensing electrodes 125. For example, leakage of stomal effluent progressing from the center opening 18 outward into or along the adhesive layer 12 may cause electrical resistance between a pair of sensing electrodes 125 to decrease. The control unit 50 may detect the decrease in electrical resistance and determine that a leak is occurring based on the decreased electrical resistance. In an embodiment, the control unit 50, via the controller, may be configured to determine a location of the leak based on the location of the sensing electrodes 125 that detected the change in resistance. The control unit 50 may be further configured to provide a notification or alert indicating that a leak has been detected and/or a location of the leak. The notification or alert may be, for example, an audible, visible, or haptic alert, or a combination thereof. The control unit 50 may also be configured for wired and/or wireless communication with other electronic devices, such as a smart phone and the like. In an embodiment, the control unit 50 may be paired, synced, or otherwise communicatively connected to the personal notification device with a known pairing or syncing operation, which may be initiated, for example, by operation of a switch.

In the present disclosure, the words "a" or "an" are to be taken to include both the singular and the plural. Conversely, any reference to plural items shall, where appropriate, include the singular. In additions, various features described with respect to any of the embodiments above may be used together, implemented in, or replace features in any of the other embodiments described above.

The disclosure is intended to cover by the appended claims all such modifications as fall within the scope of the claims.

## Claims

1. A sensor device (10) for an ostomy appliance comprising:
a sensor layer (14, 114) comprising a substrate (26, 126), a generally ring-shaped sensor region, a connector region, a tail region connecting the sensor region and the connector region, a plurality of sensing electrodes (125) arranged on a proximal side of the substrate in the sensor region, a plurality of connection points (134) arranged in the connector region and configured to electrically connect the sensor device to a control unit (50), comprising a plurality of connecting traces (127) arranged on a distal side of the substrate, wherein each of the plurality of the sensing electrodes is electrically connected to at least one of the plurality of the connection points by at least one of the plurality of connecting traces; and
a skin barrier layer (16) arranged adjacent the plurality of sensing electrodes for attaching the sensor device to a user;
wherein the sensor device is configured to detect an ostomy effluent leakage;
**characterised in that** the connecting traces are non-sensing portions of electrically conductive circuitry.

2. The sensor device (10) of claim 1, wherein each of the plurality of sensing electrodes (125) is electrically connected to at least one of the plurality of the connecting traces (127) through a via (140).

3. The sensor device (10) of any one of claims 1-2, wherein the plurality of connection points (134) are arranged on the proximal side of the substrate, wherein each of the plurality of connecting traces (127) is electrically connected to at least one of the plurality of connection points through a via (140).

4. The sensor device (10) of any one of claims 2-3, wherein the via (140) is formed from an electrically conductive material and extends through a thickness of the substrate.

5. The sensor device (10) of any one of claim 1-4, wherein the plurality of sensing electrodes (125) comprise a first sensing electrode (125A), a second sensing electrode (125B), and a third sensing electrode (125C), wherein the plurality of the connecting traces comprise a first connecting trace (127A), a second connecting trace (127B), and a third connecting trace (127C), and wherein the plurality of connection points comprise a first connection point (134A), a second connection point (134B), and a third connection point (134C).

6. The sensor device (10) of claim 5, wherein the first sensing electrode (125A) is electrically connected to the first connecting trace (127A) through a first via (140A), the second sensing electrode (125B) is electrically connected to the second connecting trace (127B) through a second via (140B), and the third sensing electrode (125C) is electrically connected to the third connecting trace (127C) through a third via (140C), wherein the first connecting trace (127A) is electrically connected to the first connection point (134A) through a fourth via (140D), the second connecting trace (127B) is electrically connected to the second connection point (134B) through a fifth via (140E), and the third connecting trace is electrically connected to the third connection point through a sixth via.

7. The sensor device (10) of claim 5, wherein the first sensing electrode is electrically connected to the first connecting trace through a first via (140A), the second sensing electrode is electrically connected to the second connecting trace through a second via (140B), the third sensing electrode is electrically connected to the second connecting trace through a third via (140C), wherein the first connecting trace is electrically connected to the first connection point through a fourth via (140D) and the second connecting trace is electrically connected to the second connection point through a fifth via (140E).

8. The sensor device (10) of claim 5, wherein the first, second, and third sensing electrodes (125A, 125B, 125C) are substantially circular electrodes, wherein each of the first, second, and third sensing electrodes are arranged at a different radial distance from a center opening of the sensor device, wherein the first, second, and third sensing electrodes are configured to determine a radial distance of an ostomy effluent leakage location.

9. The sensor device (10) of claim 5, wherein the first, second, and third sensing electrodes (125A, 125B, 125C) are arc shaped electrodes, wherein each of the first, second, and third sensing electrodes is arranged in a different section of the sensor region, wherein the first, second, and third sensing electrodes are configured to determine a location of ostomy effluent leakage.

10. The sensor device (10) of claim 9, wherein the plurality of sensing electrodes further comprise a fourth sensing electrode (125D) having an arc shape, wherein the first sensing electrode is arranged in a first quadrant of the sensor region (128), the second sensing electrode is arranged in a second quadrant of the sensor region (128), the third sensing electrode is arranged in a third quadrant of the sensor region (128), and the fourth sensing electrode is arranged in a fourth quadrant of the sensor region (128), wherein the sensor device is configured to determine a location of ostomy effluent leakage based on a location of a sensing electrode that detected a change in an electrical resistance.

11. The sensor device (10) of claim 10, wherein the plurality of connecting traces (127) further includes a fourth connecting trace (127D), wherein the first sensing electrode (125A) is arranged on the proximal side of the substrate in the first quadrant of the sensor region (128) and is electrically connected to the first connecting trace (127A) through a first via (140A), the first connecting trace extending along the fourth quadrant of the sensor region and along the tail region on the distal side of the substrate, wherein the second sensing electrode (125B) arranged on the proximal side of the substrate in the second quadrant of the sensor region is electrically connected to the second connecting trace (127B) through a second via (140B), the second connecting trace extending along the third quadrant of the sensor region and along the tail region on the distal side of the substrate, wherein the third sensing electrode (125C) arranged on the proximal side of the substrate in the third quadrant of the sensor region is electrically connected to the third connecting trace (127C) through a third via (140C), the third connecting trace extending from a lower portion of the sensor region and along the tail region on the distal side of the substrate, and wherein the fourth sensing electrode (125D) arranged on the proximal side of the substrate in the fourth quadrant of the sensor region is electrically connected to the fourth connecting trace (127D) through a fourth via (140D), the fourth connecting trace extending from a lower portion of the sensor region and along the tail region on the distal side of the substrate.

12. The sensor device (10) of claim 11, wherein the plurality of connection points (134) are arranged on the proximal side of the substrate and further comprise a fourth connection point (134D), wherein the first connecting trace (127A) is electrically connected to the first connection point (134A) via a fifth via (140E), the second connecting trace (127B) is electrically connected to the second connection point (134B) via a sixth via (140F), the third connecting trace (127C) is electrically connected to the third connection point (134C) via a seventh via (140G), and the fourth connecting trace (127D) is electrically connected to the fourth connection point (134D) via an eighth via (140H).

13. The sensor device (10) of any of claims 1-12, wherein the sensor device is configured to detect an ostomy effluent leakage based on a change in electrical resistance measured by the plurality of sensing electrodes (125).

## Patentansprüche

1. Sensorvorrichtung (10) für eine Stomaeinrichtung, umfassend:
eine Sensorschicht (14, 114), umfassend ein Substrat (26, 126), einen im Allgemeinen ringförmigen Sensorbereich, einen Verbinderbereich, einen Endbereich, der den Sensorbereich und Verbinderbereich verbindet, eine Vielzahl von Messelektroden (125), die an einer proximalen Seite des Substrats in dem Sensorbereich angeordnet ist, eine Vielzahl von Verbindungspunkten (134), die in dem Verbinderbereich angeordnet und konfiguriert ist, um die Sensorvorrichtung elektrisch mit einer Steuereinheit (50) zu verbinden, umfassend eine Vielzahl von Verbindungsspuren (127), die an einer distalen Seite des Substrats angeordnet ist, wobei jede der Vielzahl der Messelektroden über mindestens eine der Vielzahl von Verbindungsspuren elektrisch mit mindestens einem der Vielzahl der Verbindungspunkte verbunden ist; und
eine Hautbarriereschicht (16), die benachbart zu der Vielzahl von Messelektroden angeordnet ist, um die Sensorvorrichtung an einem Benutzer zu befestigen;
wobei die Sensorvorrichtung konfiguriert ist, um ein Austreten von Stomaausfluss zu erkennen;
**dadurch gekennzeichnet, dass** die Verbindungsspuren nicht als Sensor wirkende Abschnitte einer elektrisch leitfähigen Schaltung sind.

2. Sensorvorrichtung (10) nach Anspruch 1, wobei jede der Vielzahl von Messelektroden (125) über eine Durchkontaktierung (140) mit mindestens einer der Vielzahl der Verbindungsspuren (127) elektrisch verbunden ist.

3. Sensorvorrichtung (10) nach einem der Ansprüche 1 bis 2, wobei die Vielzahl von Verbindungspunkten (134) an der proximalen Seite des Substrats angeordnet ist, wobei jede der Vielzahl von Verbindungsspuren (127) über eine Durchkontaktierung (140) mit mindestens einem der Vielzahl von Verbindungspunkten elektrisch verbunden ist.

4. Sensorvorrichtung (10) nach einem der Ansprüche 2 bis 3, wobei die Durchkontaktierung (140) aus einem elektrisch leitfähigen Material ausgebildet ist und sich durch eine Dicke des Substrats erstreckt.

5. Sensorvorrichtung (10) nach einem der Ansprüche 1 bis 4, wobei die Vielzahl von Messelektroden (125) eine erste Messelektrode (125A), eine zweite Messelektrode (125B) und eine dritte Messelektrode (125C) umfasst, wobei die Vielzahl der Verbindungsspuren eine erste Verbindungsspur (127A), eine zweite Verbindungsspur (127B) und eine dritte Verbindungsspur (127C) umfasst und wobei die Vielzahl von Verbindungspunkten einen ersten Verbindungspunkt (134A), einen zweiten Verbindungspunkt (134B) und einen dritten Verbindungspunkt (134C) umfasst.

6. Sensorvorrichtung (10) nach Anspruch 5, wobei die erste Messelektrode (125A) über eine erste Durchkontaktierung (140A) elektrisch mit der ersten Verbindungsspur (127A) verbunden ist, die zweite Messelektrode (125B) über eine zweite Durchkontaktierung (140B) elektrisch mit der zweiten Verbindungsspur (127B) verbunden ist und die dritte Messelektrode (125C) über eine dritte Durchkontaktierung (140C) elektrisch mit der dritten Verbindungsspur (127C) verbunden ist, wobei die erste Verbindungsspur (127A) über eine vierte Durchkontaktierung (140D) elektrisch mit dem ersten Verbindungspunkt (134A) verbunden ist, die zweite Verbindungsspur (127B) über eine fünfte Durchkontaktierung (140E) elektrisch mit dem zweiten Verbindungspunkt (134B) verbunden ist und die dritte Verbindungsspur über eine sechste Durchkontaktierung elektrisch mit dem dritten Verbindungspunkt verbunden ist.

7. Sensorvorrichtung (10) nach Anspruch 5, wobei die erste Messelektrode über eine erste Durchkontaktierung (140A) elektrisch mit der ersten Verbindungsspur verbunden ist, die zweite Messelektrode über eine zweite Durchkontaktierung (140B) elektrisch mit der zweiten Verbindungsspur verbunden ist, die dritte Messelektrode über eine dritte Durchkontaktierung (140C) elektrisch mit der zweiten Verbindungsspur verbunden ist, wobei die erste Verbindungsspur über eine vierte Durchkontaktierung (140D) elektrisch mit dem ersten Verbindungspunkt verbunden ist und die zweite Verbindungsspur über eine fünfte Durchkontaktierung (140E) elektrisch mit dem zweiten Verbindungspunkt verbunden ist.

8. Sensorvorrichtung (10) nach Anspruch 5, wobei die erste, zweite und dritte Messelektrode (125A, 125B, 125C) im Wesentlichen kreisförmige Elektroden sind, wobei jede von der ersten, zweiten und dritten Messelektrode in einem anderen radialen Abstand von einer zentralen Öffnung der Sensorvorrichtung angeordnet ist, wobei die erste, zweite und dritte Messelektrode konfiguriert sind, um einen radialen Abstand von einer Stelle des Austretens von Stomaausfluss zu bestimmen.

9. Sensorvorrichtung (10) nach Anspruch 5, wobei die erste, zweite und dritte Messelektrode (125A, 125B, 125C) bogenförmige Elektroden sind, wobei jede von der ersten, zweiten und dritten Messelektrode in einem anderen Teilabschnitt des Sensorbereichs angeordnet ist, wobei die erste, zweite und dritte Messelektrode konfiguriert sind, um eine Stelle des Austretens von Stomaausfluss zu bestimmen.

10. Sensorvorrichtung (10) nach Anspruch 9, wobei die Vielzahl von Messelektroden ferner eine vierte Messelektrode (125D), die eine Bogenform aufweist, umfasst, wobei die erste Messelektrode in einem ersten Quadranten des Sensorbereichs (128) angeordnet ist, die zweite Messelektrode in einem zweiten Quadranten des Sensorbereichs (128) angeordnet ist, die dritte Messelektrode in einem dritten Quadranten des Sensorbereichs (128) angeordnet ist und die vierte Messelektrode in einem vierten Quadranten des Sensorbereichs (128) angeordnet ist, wobei die Sensorvorrichtung konfiguriert ist, um eine Stelle des Austretens von Stomaausfluss basierend auf einer Stelle einer Messelektrode, die eine Änderung eines elektrischen Widerstands erkannt hat, zu bestimmen.

11. Sensorvorrichtung (10) nach Anspruch 10, wobei die Vielzahl von Verbindungsspuren (127) ferner eine vierte Verbindungsspur (127D) beinhaltet, wobei die erste Messelektrode (125A) an der proximalen Seite des Substrats in dem ersten Quadranten des Sensorbereichs (128) angeordnet und über eine erste Durchkontaktierung (140A) elektrisch mit der ersten Verbindungsspur (127A) verbunden ist, wobei sich die erste Verbindungsspur entlang des vierten Quadranten des Sensorbereichs und entlang des Endbereichs an der distalen Seite des Substrats erstreckt, wobei die zweite Messelektrode (125B), die an der proximalen Seite des Substrats in dem zweiten Quadranten des Sensorbereichs angeordnet ist, über eine zweite Durchkontaktierung (140B) elektrisch mit der zweiten Verbindungsspur (127B) verbunden ist, wobei sich die zweite Verbindungsspur entlang des dritten Quadranten des Sensorbereichs und entlang des Endbereichs an der distalen Seite des Substrats erstreckt, wobei die dritte Messelektrode (125C), die an der proximalen Seite des Substrats in dem dritten Quadranten des Sensorbereichs angeordnet ist, über eine dritte Durchkontaktierung (140C) elektrisch mit der dritten Verbindungsspur (127C) verbunden ist, wobei sich die dritte Verbindungsspur von einem unteren Abschnitt des Sensorbereichs und entlang des Endbereichs an der distalen Seite des Substrats erstreckt, und wobei die vierte Messelektrode (125D), die an der proximalen Seite des Substrats in dem vierten Quadranten des Sensorbereichs angeordnet ist, über eine vierte Durchkontaktierung (140D) elektrisch mit der vierten Verbindungsspur (127D) verbunden ist, wobei sich die vierte Verbindungsspur von einem unteren Abschnitt des Sensorbereichs und entlang des Endbereichs an der distalen Seite des Substrats erstreckt.

12. Sensorvorrichtung (10) nach Anspruch 11, wobei die Vielzahl von Verbindungspunkten (134) an der proximalen Seite des Substrats angeordnet ist und ferner einen vierten Verbindungspunkt (134D) umfasst, wobei die erste Verbindungsspur (127A) über eine fünfte Durchkontaktierung (140E) elektrisch mit dem ersten Verbindungspunkt (134A) verbunden ist, die zweite Verbindungsspur (127B) über eine sechste Durchkontaktierung (140F) elektrisch mit dem zweiten Verbindungspunkt (134B) verbunden ist, die dritte Verbindungsspur (127C) über eine siebte Durchkontaktierung (140G) elektrisch mit dem dritten Verbindungspunkt (134C) verbunden ist und die vierte Verbindungsspur (127D) über eine achte Durchkontaktierung (140H) elektrisch mit dem vierten Verbindungspunkt (134D) verbunden ist.

13. Sensorvorrichtung (10) nach einem der Ansprüche 1 bis 12, wobei die Sensorvorrichtung konfiguriert ist, um ein Austreten von Stomaausfluss basierend auf einer Änderung des elektrischen Widerstands, die durch die Vielzahl von Messelektroden (125) gemessen wird, zu erkennen.

## Revendications

1. Dispositif de capteur (10) pour un appareillage de stomie comprenant :
une couche de capteur (14, 114) comprenant un substrat (26, 126), une région de capteur généralement annulaire, une région de connexion, une région de queue connectant la région de capteur et la région de connexion, une pluralité d'électrodes de détection (125) agencées sur une face proximale du substrat dans la région de capteur, une pluralité de points de connexion (134) agencés dans la région de connexion et configurés pour connecter électriquement le dispositif de capteur à une unité de commande (50), comprenant une pluralité de pistes de connexion (127) agencées sur une face distale du substrat, dans lequel chacune de la pluralité d'électrodes de détection est connectée électriquement à au moins un de la pluralité de points de connexion par au moins une de la pluralité de pistes de connexion ; et
une couche barrière cutanée (16) agencée à adjacente à la pluralité d'électrodes de détection pour fixer le dispositif de capteur à un utilisateur ;
dans lequel le dispositif de capteur est configuré pour détecter une fuite d'effluent de stomie ;
**caractérisé en ce que** les pistes de connexion sont des parties de non-détection de circuits électriquement conducteurs.

2. Dispositif de capteur (10) selon la revendication 1, dans lequel chacune de la pluralité d'électrodes de détection (125) est connectée électriquement à au moins une de la pluralité de pistes de connexion (127) à travers un via (140).

3. Dispositif de capteur (10) selon l'une quelconque des revendications 1 à 2, dans lequel la pluralité de points de connexion (134) sont agencés sur la face proximale du substrat, dans lequel chacune de la pluralité de pistes de connexion (127) est connectée électriquement à au moins un de la pluralité de points de connexion à travers un via (140).

4. Dispositif de capteur (10) selon l'une quelconque des revendications 2 à 3, dans lequel le via (140) est formé à partir d'un matériau électriquement conducteur et s'étend à travers une épaisseur du substrat.

5. Dispositif de capteur (10) selon l'une quelconque des revendications 1 à 4, dans lequel la pluralité d'électrodes de détection (125) comprennent une première électrode de détection (125A), une deuxième électrode de détection (125B) et une troisième électrode de détection (125C), dans lequel la pluralité de pistes de connexion comprennent une première piste de connexion (127A), une deuxième piste de connexion (127B) et une troisième piste de connexion (127C), et dans lequel la pluralité de points de connexion comprennent un premier point de connexion (134A), un deuxième point de connexion (134B) et un troisième point de connexion (134C).

6. Dispositif de capteur (10) selon la revendication 5, dans lequel la première électrode de détection (125A) est connectée électriquement à la première piste de connexion (127A) à travers un premier via (140A), la deuxième électrode de détection (125B) est connectée électriquement à la deuxième piste de connexion (127B) à travers un deuxième via (140B), et la troisième électrode de détection (125C) est connectée électriquement à la troisième piste de connexion (127C) à travers un troisième via (140C), dans lequel la première piste de connexion (127A) est connectée électriquement au premier point de connexion (134A) à travers un quatrième via (140D), la deuxième piste de connexion (127B) est connectée électriquement au deuxième point de connexion (134B) à travers un cinquième via (140E) et la troisième piste de connexion est connectée électriquement au troisième point de connexion à travers un sixième via.

7. Dispositif de capteur (10) selon la revendication 5, dans lequel la première électrode de détection est connectée électriquement à la première piste de connexion à travers un premier via (140A), la deuxième électrode de détection est connectée électriquement à la deuxième piste de connexion à travers un deuxième via (140B), la troisième électrode de détection est connectée électriquement à la deuxième piste de connexion à travers un troisième via (140C), dans lequel la première piste de connexion est connectée électriquement au premier point de connexion à travers un quatrième via (140D) et la deuxième piste de connexion est connectée électriquement au deuxième point de connexion à travers un cinquième via (140E).

8. Dispositif de capteur (10) selon la revendication 5, dans lequel les première, deuxième et troisième électrodes de détection (125A, 125B, 125C) sont des électrodes sensiblement circulaires, dans lequel chacune des première, deuxième et troisième électrodes de détection est agencée à une distance radiale différente d'une ouverture centrale du dispositif de capteur, dans lequel les première, deuxième et troisième électrodes de détection sont configurées pour déterminer une distance radiale d'un emplacement de fuite d'effluent d'ostomie.

9. Dispositif de capteur (10) selon la revendication 5, dans lequel les première, deuxième et troisième électrodes de détection (125A, 125B, 125C) sont des électrodes en forme d'arc, dans lequel chacune des première, deuxième et troisième électrodes de détection est agencée dans une section différente de la région de capteur, dans lequel les première, deuxième et troisième électrodes de détection sont configurées pour déterminer un emplacement de fuite d'effluent d'ostomie.

10. Dispositif de capteur (10) selon la revendication 9, dans lequel la pluralité d'électrodes de détection comprennent en outre une quatrième électrode de détection (125D) ayant une forme d'arc, dans lequel la première électrode de détection est agencée dans un premier quadrant de la région de capteur (128), la deuxième électrode de détection est agencée dans un deuxième quadrant de la région de capteur (128), la troisième électrode de détection est agencée dans un troisième quadrant de la région de capteur (128) et la quatrième électrode de détection est agencée dans un quatrième quadrant de la région de capteur (128), dans lequel le dispositif de capteur est configuré pour déterminer un emplacement de fuite d'effluent d'ostomie sur la base d'un emplacement d'une électrode de détection qui a détecté un changement de résistance électrique.

11. Dispositif de capteur (10) selon la revendication 10, dans lequel la pluralité de pistes de connexion (127) comporte en outre une quatrième piste de connexion (127D), dans lequel la première électrode de détection (125A) est agencée sur la face proximale du substrat dans le premier quadrant de la région de capteur (128) et est connectée électriquement à la première piste de connexion (127A) à travers un premier via (140A), la première piste de connexion s'étendant le long du quatrième quadrant de la région de capteur et le long de la région de queue sur la face distale du substrat, dans lequel la deuxième électrode de détection (125B), agencée sur la face proximale du substrat dans le deuxième quadrant de la région de capteur, est connectée électriquement à la deuxième piste de connexion (127B) à travers un deuxième via (140B), la deuxième piste de connexion s'étendant le long du troisième quadrant de la région de capteur et le long de la région de queue sur la face distale du substrat, dans lequel la troisième électrode de détection (125C), agencée sur la face proximale du substrat dans le troisième quadrant de la région de capteur, est connectée électriquement à la troisième piste de connexion (127C) à travers un troisième via (140C), la troisième piste de connexion s'étendant à partir d'une partie inférieure de la région de capteur et le long de la région de queue sur la face distale du substrat, et dans lequel la quatrième électrode de détection (125D), agencée sur la face proximale du substrat dans le quatrième quadrant de la région de capteur, est connectée électriquement à la quatrième piste de connexion (127D) à travers un quatrième via (140D), la quatrième piste de connexion s'étendant à partir d'une partie inférieure de la région de capteur et le long de la région de queue sur la face distale du substrat.

12. Dispositif de capteur (10) selon la revendication 11, dans lequel la pluralité de points de connexion (134) sont agencés sur la face proximale du substrat et comprennent en outre un quatrième point de connexion (134D), dans lequel la première piste de connexion (127A) est connectée électriquement au premier point de connexion (134A) à travers un cinquième via (140E), la deuxième piste de connexion (127B) est connectée électriquement au deuxième point de connexion (134B) à travers un sixième via (140F), la troisième piste de connexion (127C) est connectée électriquement au troisième point de connexion (134C) à travers un septième via (140G) et la quatrième piste de connexion (127D) est connectée électriquement au quatrième point de connexion (134D) à travers un huitième via (140H).

13. Dispositif de capteur (10) selon l'une quelconque des revendications 1 à 12, dans lequel le dispositif de capteur est configuré pour détecter une fuite d'effluent d'ostomie sur la base d'un changement de résistance électrique mesuré par la pluralité d'électrodes de détection (125).
